# FASCICULE DE BREVET EUROPEEN

(11) **EP 0 429 324 B1**
(45) Date de publication et mention de la délivrance du brevet: **27.03.1996**
(21) Numéro de dépôt: 90403060.8
(22) Date de dépôt: 30.10.1990
(51) Int. Cl.: A61K 31/44

(54) **Application du N-méthyl (pyridyl-3)-2 tétrahydrothiopyrannecarbothioamide-2-oxyde-1-(1R,2R) à la préparation de médicaments destinés au traitement de l'insuffisance coronarienne**
Verwendung von (1R,2R)-N-Methyl-2-(3-pyridyl)-2-tetrahydrothiopyrancarbothioamid-1-Oxid zur Herstellung von Arzneimitteln zur Behandlung von Koronarschwäche
Use of (1R,2R)-N-methyl-2-(3-pyridyl)2-tetrahydrothiopyran-carbothioamide-1-oxide for the preparation of medicaments for the treatment of coronary insufficiency

(30) Priorité: 31.10.1989 FR 8914272
(43) Date de publication de la demande: 29.05.1991
(73) Titulaire: RHONE-POULENC SANTE, F-92160 Antony (FR)
(72) Inventeur: Cavero, Icilio, F-94000 Creteil (FR); Mondot, Serge, F-06800 Cagnes sur Mer (FR)
(74) Mandataire: Pilard, Jacques

(56) Documents cités:
- EP-A- 0 097 584
- EP-A- 0 323 745
- PFLÜGERS ARCH. EUR. J. PHYSIOL., vol. 414, no. 6, septembre 1989, pages 669-675; D. ESCANDE et al.: "Potassium channel openers act through an activation of ATP-sensitive K+ channels in guinea-pig cardiac myocytes"
- PFLÜGERS ARCH. EUR. J. PHYSIOL., vol. 414, suppl. 1, 1989, page S175, Springer Verlag; D. THURINGER et al.: "The potassium channel opener RP 49356 modifies the ATP-sensitivity of K+-ATP channels in cardiac myocytes", & SYMPOSIUM ON POTASSIUM CHANNELS: FUNCTION, REGULATION AND PHARMACOLOGY IN NEURONAL, MUSCULAR, SECRETORY AND IMMUNE SYSTEMS, HELD AT THE 6TH INTERNATIONAL RHONE-POULENC SANTE FOUNDATION ROUND TABLE, PARIS, FRANCE, 23 JANVIER 1989
- BR. J. PHARMACOL., vol. 95, suppl., 1988, page 814P; D. ESCANDE et al.: "RP 49356 is a potent opener of ATP-modulated potassium channels in cardiac myocytes"
- J. AUTON. PHARMAC., vol. 9, no. 5, octobre 1989, pages 329-336; K. LAWSON et al.: "Effects of Ca2+ antagonists and K+-channel activators on K+-induced contractions in the rat aorta"
- BR. J. PHARMACOLOGY, vol. 95, suppl., 1988, page 813P; S. MONDOT et al.: "EP 49356: A vasorelaxant agent with potassium channel activating properties"
- BIOCHEMICAL AND BIOPHYSICAL RESEARCH COMMUNICATIONS, vol. 154, no. 2, 29 juillet 1988, pages 620-625, Academic Press., Inc.; D. ESCANDE et al.: "The potassium channel opener cromakalim (BRL 34915) activates ATP-dependent K+ channels in isolated cardiac myocytes"
- EUROPEAN JOURNAL OF PHARMACOLOGY, vol. 165, no. 2-3, 20 juin 1989, pages 231-239, Elsevier Science Publishers B.V. (Biomedical Division); M. ELTZE: "Glibenclamide is a competitive antagonist of cromakalim, pinacidil and RP 49356 in guinea-pig pulmonary artery"
- Circulation Research, 70(2), 1992, pp. 223-233

## Description

La présente invention concerne l'application de (pyridyl-3)-2 tétrahydrothiopyrannecarbothioamide-2 oxyde-1-(1R,2R) à la préparation de médicaments efficaces en thérapie de l'insuffisance coronarienne, dans le traitement de l'angine de poitrine et comme protecteur cardiaque.

Dans le brevet européen EP 0 097 584 ont été décrits des dérivés du thioformamide de formule générale : 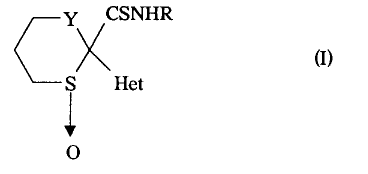
dans laquelle R représente un atome d'hydrogène ou un radical alcoyle contenant 1 à 4 atomes de carbone, Het représente un radical hétérocyclique à caractère aromatique et Y représente une liaison de valence ou un radical méthylène, qui présentent des propriétés antihypertensives remarquables.

Les articles de D. Escande et coll., Pflügers Arch. Eur. J. Physiol., 414 (6) 669-675 (1989), de S. Mondot et coll., Brit. J. Pharmacol., 95, supp., 813P (1988) et de D. Escande et coll., Brit. J. Pharmacol., 95, supp., 814P (1988) décrivent l'activité du RP 49356 sur les cellules myocardiques isolées de cobayes étudiées par la technique du patch-champ membranaire, montrant que le RP 49356 est un activateur de canaux potassiques, ainsi que les activités vasorelaxante et antihypertensive potentielles du RP 49356.

K. Lawson et I. Cavero, J. Auton. Pharmac., 9, 329-336 (1989) enseignent une approche fonctionnelle à la distinction qualitative et quantitative des activateurs de canaux potassiques des autres catégories de produits.

D. Escande et coll., Biochem. Biophys. Res. Comm., 154, 620-625 (1988) tentent d'expliquer l'activité de la cromakalim et du pinacidil, qui produisent un effet vasodilatateur et antihypertenseur, en relation avec l'activation des canaux potassiques.

Enfin M. Eltze, Eur. J. Pharmacol., 165, 231-239 (1989) enseigne l'action antagoniste du glibenclamide vis-à-vis de la cormakalim, du pinacidil et du RP 49356.

Il a maintenant été trouvé, et c'est ce qui fait l'objet de la présente invention, que le N-méthyl (pyridyl-3)-2 tétrahydrothiopyrannecarbothioamide-2 oxyde-1 sous forme de son 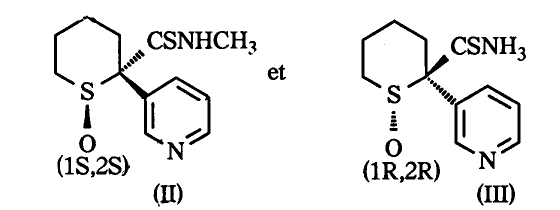
isomère (III) dont la configuration absolue est 1R,2R présente des propriétés coronarodilatatrices intéressantes.

La présente invention a donc pour objet l'application de l'isomère (III) du N-méthyl (pyridyl-3)-2 tétrahydrothiopyrannecarbothioamide-2 oxyde-1 à la préparation de médicaments à action myorelaxante vasculaire particulièrement utiles comme protecteurs vis-à-vis de l'ischémie myocardique notamment dans le traitement de l'angine de poitrine.

L'influence du mélange des produits de formules (II) et (III) (mélange racémique) et du produit de formule (II) sur la circulation artérielle coronaire peut être mise en évidence de la manière suivante:

Des chiens de race Beagle, mâles ou femelles, d'un poids compris entre 14 et 18 kg sont anesthésiés au pentobarbital sodique (35 mg/kg i.v. suivie d'une perfusion continue de 3 mg/kg/heure sous un débit de 0,2 cm3/minute).

Les animaux sont intubés avec une sonde endotrachéale et placés sous respiration artificielle. Une thoracotomie gauche est pratiquée au niveau du 5ème espace intercostal et une sonde électromagnétique est placée sur l'origine de l'artère circonflexe ainsi que sur la crosse aortique pour l'enregistrement du débit artériel coronaire et du débit cardiaque.

La pression artérielle est mesurée à l'aide d'un capteur STATAM P23Id relié à un cathéter introduit dans l'artère fémorale. La fréquence cardiaque est calculée électrouiquement à partir de l'onde puisatile. Une veine fémorale est cathétérisée pour l'injection des produits.

Tous les paramètres sont enregistrés sur un Polygraph 8 pistes (Linearcorder Mark VII) et leurs valeurs, après digitalisation, sont stockées dans un computer pour traitement des données.

Les produits à étudier, en solution dans de l'eau distillée, sont administrés en perfusion intraveineuse pendant 15 minutes sous un volume et à la vitesse de 1 cm3/minute.

Le mélange des isomères (II) et (III) (mélange racémique) aux doses de 2,5 et 5 µg/kg/minute perfusées pendant 15 minutes (doses totales administrées égales à 37,5 et 75 µg/kg à raison de 3 chiens traités par dose) augmente de façon nette le débit artériel coronaire; l'effet coronarodilatateur atteint son acmé d'effet en fin de perfusion (respectivement +49 % et +112 % pour des débits initiaux de 33 ± 2 et 38 ± 4 cm3/minute) et il persiste encore de façon significative pendant environ 1 heure après la fin de la perfusion.

L'isomère (III) entraîne également une augmentation nette et durable du débit artériel coronaire et ce, avec une puissance d'activité environ 2 fois plus élevée que celle du mélange racémique : en fin de perfusion, à l'acmé d'effet on observe une augmentation du débit artériel coronaire de respectivement +62 % et +103 % aux doses respectives de 1 et 2,5 µg/kg (doses totales administrées égales à 15 et 37,5 µg/kg).

Il est tout particulièrement intéressant de noter que l'effet coronarodilatateur n'est accompagné que d'une faible accélération (environ 10 %) de la fréquence cardiaque ainsi que d'une réduction très modérée (voisine de 10-12 %) de la pression artérielle moyenne. Ces résultats, qui sont rassemblés dans le tableau I, indiquent que ces produits exercent un effet myorelaxant vasculaire qui est sélectif pour le lit artériel coronaire puisqu'il n'est pas observé au niveau de la circulation systémique (absence d'hypotension et d'augmentation du débit cardiaque).

**TABLEAU I**

| EFFETS SUR LA FREQUENCE CARDIAQUE (FC), LA PRESSION ARTERIELLE MOYENNE (PAM) ET LE DEBIT ARTERIEL CORONAIRE (DAC) CHEZ LE CHIEN ANESTHESIE AU PENTOBARBITAL | | | | | | | | | | |
|---|---|---|---|---|---|---|---|---|---|---|
| Produits | Dose µg/kg/mn i.v. | Paramètres (a) | Valeur initiale moyenne ± ESM | Variations moyennes (%) par rapport aux valeurs initiales | | | | | | |
| | | | | Après début de perfusion i.v. (minutes) | | | Après fin de perfusion i.v. (minutes) | | | |
| | | | | 5 | 10 | 15 | 5 | 15 | 30 | 60 |
| Mélange racémique des isomères (II) et (III) | 2.5 | FC | 158 ±4 | + 1 | + 3 | + 7 | + 6 | 0 | 0 | - 1 |
| | | PAM | 106 ±4 | - 5 | - 9 | - 10 | - 11 | - 11 | - 9 | - 5 |
| | | DAC | 33 ±2 | + 15 | + 41 | + 49 | + 34 | + 12 | + 14 | + 18 |
| | 5.0 | FC | 155 ± 3 | + 3 | + 9 | + 10 | + 6 | + 8 | + 5 | + 2 |
| | | PAM | 100 ± 2 | - 4 | - 11 | - 12 | - 12 | - 11 | - 10 | - 8 |
| | | DAC | 38 ± 4 | + 49 | +103 | +112 | + 86 | + 34 | + 19 | 0 |
| Isomère II I | 1.0 | FC | 149 ± 2 | + 3 | + 9 | + 10 | + 11 | + 13 | + 11 | + 11 |
| | | PAM | 115 ± 5 | - 1 | 0 | 0 | + 1 | + 2 | + 2 | + 8 |
| | | DAC | 39 ± 2 | + 15 | + 51 | + 62 | + 43 | + 28 | + 18 | + 18 |
| | 2.5 | FC | 159 ± 3 | + 8 | + 9 | + 10 | + 9 | + 9 | + 3 | + 3 |
| | | PAM | 118 ± 7 | + 3 | + 5 | - 10 | - 13 | - 11 | - 11 | - 11 |
| | | DAC | 39 ± 2 | + 41 | + 88 | +103 | + 88 | + 48 | + 38 | + 21 |

| | | | | | | | | | | |
|---|---|---|---|---|---|---|---|---|---|---|
| (a) FC : battements/min PAM: mmHg DAC: ml/min | | | | | | | | | | |

Il en résulte que le mélange racémique des isomères (II) et (III) et l'isomère (III) améliorent la circulation coronaire par un effet direct et spécifique sur le lit artériel sans modification notable de la dynamique cardiaque et notamment de la pression artérielle.

Chez le chien, aux doses quotidiennes de 0,3 et 1 mg/kg/jour par voie orale (1 mois de traitement) le N-méthyl (pyridyl-3)-2 tétrahydrothiopyrannecarbothioamide-2 oxyde-1 a montré une tolérance générale satisfaisante.

L'isomère (III) est utile dans la thérapeutique de l'insuffisance coronarienne et peut être utilisé en tant qu'anti-angoreux et protecteur cardiaque.

Le mélange racémique des isomères (II) et (III) du N-méthyl (pyridyl-3)-2 tétrahydrothiopyrannecarbothioamide-2 oxyde-1 peut être préparé dans les conditions décrites dans le brevet européen EP 0 097 584 dans lequel il est désigné par "Forme A" ou "produit le plus polaire" [la polarité étant déterminée par chromatographie sur couche mince (CCM)].

Le N-méthyl (pyridyl-3)-2 tétrahydrothiopyrannecarbothioamide-2 oxyde-(1R,2R) peut être obtenu soit par dédoublement du racémique dans les conditions appropriées, soit par action de l'isothiocyanate de méthyle sur un sulfoxyde de formule : 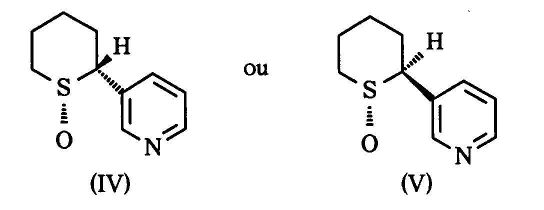
préalablement anionisé.

Généralement, la réaction est effectuée en ajoutant une solution de sulfoxyde de formule (IV) ou (V) ou d'un mélange de ces sulfoxydes dans un solvant organique inerte tel qu'un éther comme le tétrahydrofuranne à de l'amidure de sodium (éventuellement préparé in situ) dans l'ammoniac liquide en opérant à la température d'ébullition du mélange réactionnel, c'est-à-dire à -30°C, puis en ajoutant une solution d'isothiocyanate de méthyle dans un solvant organique inerte tel qu'un éther comme le tétrahydrofuranne à la même température.

Les sulfoxydes de formule (IV) ou (V) ou leurs mélanges peuvent être obtenus par oxydation stéréosélective d'un produit de formule : 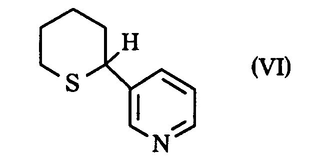
soit par voie chimique soit par voie biochimique.

L'oxydation sélective par voie chimique est réalisée en présence d'un inducteur d'asymétrie tel que le (+)-tartrate de diéthyle et d'un dérivé du titane (IV) tel qu'un alcoolate de titane (IV) au moyen d'un hydroperoxyde tel que l'hydroperoxyde de cumyle ou de tert.butyle. Généralement on opère dans un solvant orgauique tel qu'un hydrocarbure aliphatique halogéné (chlorure de méthylène, dichioro-1,2 éthane) à une température voisine de -20°C.

L'oxydation sélective par voie biochimique est réalisée au moyen d'une culture d'un champignon filamenteux tel que Aspergillus foetidus NRRL 337.

Le produit de formule (VI) peut être obtenu, par exemple, par décarboxylation de l'acide de formule : 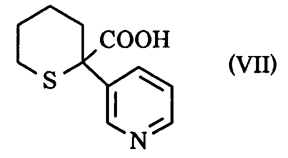
par chauffage à une température comprise entre 130 et 160°C, l'acide de formule (VII) étant obtenu dans les conditions décrites dans le brevet européen EP 0 073 704.

Les médicaments améliorant la circulation coronarienne sont constitués par le N-méthyl (pyridyl-3)-2 tétrahydrothiopyrannecarbothioamide-2 oxyde-1 sous forme de l'isomère 1R,2R à l'état pur ou sous forme d'une composition dans laquelle ils sont associés à tout autre produit pharmacologiquement compatible pouvant être inerte ou physiologiquement actif. Ces médicaments peuvent être utilisés par voie orale, intraveineuse, sublinguale ainsi que par voie transdermale.

Comme compositions solides pour administration orale ou sublinguale peuvent être utilisés des comprimés, des pilules, des poudres (capsules de gélatine, cachets) ou granulés. Dans ces compositions le principe actif est mélangé à un ou plusieurs diluants inertes tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables par exemple l'oléate d'éthyle ou autres solvants organiques convenables. Ces compositions peuvent également contenir des adjuvants, en particulier des agents mouillants, isotonisants, émulsifiants, dispersants et stabilisants.

La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration transdermale se présentent sous forme de patches dans lesquels le médicament est dispersé dans une matrice convenable permettant sa libération progressive.

En thérapeutique humaine, le N-méthyl (pyridyl-3)-2 tétrahydrothiopyrannecarbothioamide-2 oxyde sous forme de l'isomère 1R,2R est utile dans le traitement des insuffisances coronariennes et notamment dans le traitement de l'angine de poitrine. Il est également utile comme protecteur cardiaque vis-à-vis d'attaques ischémiques.

Les doses dépendent de l'effet recherché, de la durée du traitement et de la voie d'administration utilisée. Pour un adulte, elles sont généralement comprises entre 0,05 et 5 mg par jour par voie parentérale et entre 1 et 10 mg par jour par voie orale. D'une façon générale, le médecin déterminera la posologie appropriée en fonction de l'âge, du poids et de tous les autres facteurs propres au sujet à traiter.

Les exemples suivants illustrent des compositions pharmaceutiques utilisables pour le traitement de l'insuffisance coronarienne.

### EXEMPLE

On prépare selon la technique habituelle une solution injectable par voie intraveineuse ayant la composition suivante :

| | |
|---|---|
| - N-méthyl (pyridyl-3)-2 tétrahydrothiopyrannecarbothioamide-2 oxyde-1-(1R,2R) | 1 mg |
| - soluté injectable | 5 cm3 |

## Revendications

1. Application du N-méthyl-(pyridyl-3)-2 tétrahydrothiopyrannecarbothioamide-2 oxyde-1-(1R,2R) à la préparation d'un médicament efficace en thérapie de l'insuffisance coronarienne.

2. Application selon la revendication 1 caractérisée en ce que le médicament est destiné au traitement de l'angine de poitrine.

3. Application selon la revendication 1 caractérisée en ce que le médicament est destiné à être utilisé comme protecteur cardiaque.

4. Application selon l'une des revendications 1 à 3 à la préparation d'un médicament utilisé à la dose maximale de 5 mg par jour pour un adulte par voie parentérale ou de 10 mg par jour pour un adulte par voie orale.

## Claims

1. Use of (1R,2R)-N-methyl-2-(3-pyridyl)-tetrahydrothiopyran-2-carbothioamide 1-oxide in the preparation of a medicinal product which is effective in the therapy of coronary insufficiency.

2. Use according to Claim 1, characterized in that the medicinal product is intended for the treatment of angina pectoris.

3. Use according to Claim 1, characterized in that the medicinal product is intended for use as a cardioprotective agent.

4. Use according to one of Claims 1 to 3 in the preparation of a medicinal product used at a maximum dose of 5 mg daily for an adult via the parenteral route or 10 mg daily for an adult via the oral route.

## Patentansprüche

1. Verwendung von (1R,2R)-N-methyl-2-(pyridyl-3)-tetrahydropyran 2-carbothioamid-1-oxyd zur Herstellung eines Arzneimittels, das in der Therapie der Coronarinsuffizienz wirksam ist.

2. Verwendung gemäß Anspruch 1, dadurch gekennzeichnet, daß das Arzneimittel zur Behandlung der Angina Pectoris bestimmt ist.

3. Verwendung gemäß Anspruch 1, dadurch gekennzeichnet, daß das Arzneimittel dazu bestimmt ist als Herzschutzmittel verwendet zu werden.

4. Verwendung gemäß einem der Ansprüche 1 bis 3 zur Herstellung eines Arzneimittels, das in einer Maximaldosis von 5 mg pro Tag für einen Erwachsenen auf parenteralem Wege oder von 10 mg pro Tag für einen Erwachsenen auf oralem Wege verwendet wird.
